(19) Europäisches Patentamt
European Patent Office
Office européen des brevets

(11) EP 0 973 524 B1

# (12) FASCICULE DE BREVET EUROPEEN

(45) Date de publication et mention de la délivrance du brevet:
**28.08.2002 Bulletin 2002/35**

(21) Numéro de dépôt: **98913857.3**

(22) Date de dépôt: **06.03.1998**

(51) Int Cl.⁷: **A61K 31/565**, A61K 31/57, A61K 7/48, A61P 17/04

(86) Numéro de dépôt international:
**PCT/FR98/00457**

(87) Numéro de publication internationale:
**WO 98/040074 (17.09.1998 Gazette 1998/37)**

(54) **UTILISATION COSMETIQUE OU DERMATOLOGIQUE DE STEROIDES 7-HYDROXYLES**

KOSMETISCHE ODER DERMATOLOGISCHE VERWENDUNG VON 7-HYDROXYLIERTEN STEROIDEN

COSMETIC OR DERMATOLOGICAL USE OF 7-HYDROXYLATED STEROIDS

(84) Etats contractants désignés:
**DE ES GB IT**

(30) Priorité: **10.03.1997 FR 9702811**

(43) Date de publication de la demande:
**26.01.2000 Bulletin 2000/04**

(73) Titulaire: **Vitasterol S.A.R.L.
75006 Paris (FR)**

(72) Inventeur: **DRAY, Fernand, Joseph
F-75006 Paris (FR)**

(74) Mandataire: **Mouget-Goniot, Claire
Cabinet Orès,
6, Avenue de Messine
75008 Paris (FR)**

(56) Documents cités:
**EP-A- 0 189 738**
**EP-A- 0 415 766**
**EP-A- 0 723 775**
**WO-A-94/08588**
**WO-A-95/10283**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 973 524 B1

## Description

La présente invention concerne l'utilisation de stéroïdes 7-hydroxylés pour la préparation de compositions cosmétiques ou dermatologiques pour prévenir et/ou traiter les effets cutanés du veillissement et de l'action d'irradiations ultra-violettes.

La formation des hormones stéroïdes, leurs interrelations et leurs fonctions ont été largement décrites dans l'art antérieur. Les fonctions de la pregnenolone (PREG) et de la déhydroépiandrostérone (DHEA) ainsi que de certains de leurs dérivés sont notamment rappelées dans la demande de brevet PCT publiée sous le numéro WO 94/08588.

La DHEA et son dérivé sulfate (S-DHEA) circulent en quantité importante chez l'homme adulte, mais son taux diminue avec l'âge (Orentreich & coll., *J. Clin. Endocr. Metab.* 59: 551-555, 1984). Il a ainsi été proposé, par exemple dans le demande de brevet français publiée sous le numéro 2 729 854 ou la demande de brevet Européen correspondante publiée sous le numéro 723 775, d'utiliser la S-DHEA dans une composition cosmétique à application topique destinée au traitement de certains signes de veillissement. De multiples effets de la DHEA ont été décrits, mais certains s'opposent aux processus et aux pathologies associées au vieillissement (Watson & coll., *Drug & Aging* 9: 274-291, 1996).

Malgré de nombreuses expérimentations, aucune des explications avancées pour les effets de la DHEA n'a pu être pleinement prouvée (Kalimi & coll., *Molec. Cell. Biochem.* 131: 99-104, 1994), et l'utilisation thérapeutique de la DHEA a révélé des effets secondaires indésirables, en particulier chez la femme, en tant que précurseur potentiel des hormones androgènes.

Il a maintenant été montré que les dérivés 7-hydroxylés de la PREG et de la DHEA sont formés par un système enzymatique présent dans de multiples tissus et organes, dont la peau, où ils favorisent les mécanismes liés à l'immunité (Morfin & Courchay, *J. Steroid Biochem. Molec. Biol.* 50: 91-100, 1994). Comme les taux de DHEA circulants, l'activité de ces enzymes hydroxylantes diminue avec l'âge (Doostzadeh & Morfin, *Steroids* 61: 613-620, 1996).

La Demanderesse s'est donc intéressée aux effets de stéroïdes 7-hydroxylés et de leurs dérivés sur les cellules qui constituent la peau humaine et qui sont affectées lors du vieillissement ou après irradiation UV. Les travaux de recherche réalisés par la Demanderesse ont permis de mettre en évidence que les effets des glucocorticoïdes conduisant à l'apoptose cellulaire sont oblitérés par les stéroïdes 7-hydroxylés et que leur action sur les cellules cutanées se traduit par des effets bénéfiques et protecteurs.

De façon surpenante, les résultats obtenus avec les composés de l'invention ne correspondent pas à ceux classiquement attendus avec des hormones stéroïdes. En effet, le processus d'hydroxylation effectué par l'organisme sur la PREG ou la DHEA est irréversible, et de ce fait les hormones stéroïdes classiques ne peuvent plus être produites à partir des dérivés 7-hydroxylés.

En conséquence, l'utilisation de 7-hydroxystéroïdes à des fins cosmétologiques pour traiter ou prévenir les effets cutanés du vieillissement présente des avantages remarquables par rapport aux stéroïdes des compositions cosmétiques de l'art antérieur.

Les travaux récents concernant les modifications cutanées provoquées par l'âge ou les UV et leur traitement médical envisagent spécifiquement l'acide rétinoïque, les $\alpha$-hydroxy acides et la DHEA, mais ne mentionnent pas les 7-hydroxystéroides (Gilchrest, *Brit. J. Dermatol.* 135: 867-875, 1996; Watson & coll., *Drugs & Aging* 9: 274-291, 1996).

La production de dérivés 7-hydroxylés de la DHEA est connue depuis longtemps, dans les tissus du foetus humain (Sulcova & coll., *Endocr. Experiment.* 2: 167-172, 1968), dans l'épithelium amniotique (Sulcova & coll., *J. Steroid Biochem.* 7: 101-104, 1976), le foie humain (Starka, *Sond. Zeit. Natur.* 17: 1-2, 1965), les testicules et l'épididym humains (Sulcova & Starka, *Experimentia* 28: 1361-1362, 1972) et dans les pré-adipocytes humains (Khalil & coll. *J. Steroid Biochem. Molec. Biol.* 46: 585-594, 1993). Par ailleurs, les taux circulants de 7$\alpha$-hydroxy-DHEA ont été mesurés chez des femmes préménopausées à 200-300 pg/ml (Skinner & coll. *Steroids* 30:315-330, 1977) et la 3$\beta$,7$\alpha$-dihydroxy-5$\alpha$-androstan-17-one (7$\alpha$-hydroxyisoandrostérone) a été caractérisée dans les urines humaines (Jacolot & coll. *J. Steroid Biochem.* 14: 663-669, 1981). Plus récemment, le phénomène de la 7-hydroxylation a été étendu à d'autres stéroïdes qui possèdent, en commun avec la DHEA, une structure 3$\beta$-hydroxylée. Il s'agit de la PREG (Akwa & coll. *Biochem. J.* 288: 959-964, 1992; Morfin & Courchay *J. Steroid Biochem. Molec. Biol.* 50: 91-100, 1994), du 5$\alpha$-androstane-3$\beta$,17$\beta$-diol (Morfin & coll. *Biochimie* 59: 637-644, 1977; Morfin & coll. *J. Steroid Biochem.* 12: 629-632, 1980), du 3$\beta$-hydroxy-5$\alpha$-androstan-17-one (Akwa & coll. *Biochem. J.* 288: 959-964, 1992) et du 3$\beta$-hydroxy-5$\alpha$-pregnan-20-one (Strömstedt & coll. *Molec. Pharmacol.* 44: 1077-1083, 1993).

Quelques travaux sur des stéroïdes 7-hydroxylés ont prouvé qu'ils étaient dénués d'effets hormonaux propres tant androgènes qu'oestrogènes ou sur la secrétion des hormones hypophysaires (Celotti & coll. *J. Steroid Biochem.* 18: 397-401, 1983; Sunde & coll. *J. Steroid Biochem.* 16: 483-488, 1982). L'ensemble de ces résultats a donc conduit à considérer la 7-hydroxylation des stéroïdes comme un processus terminal d'inactivation hormonale conduisant à l'excrétion urinaire et biliaire des stéroïdes 7-hydroxylés produits (Ofner & coll. *J. Steroid Biochem.* 11: 1367-1379, 1979; Strômstedt & coll. *Molec. Pharmacol.* 44: 1077-1083, 1993; Khalil & coll. *J. Steroid Biochem. Molec. Biol.* 48: 545-552, 1994). Ce n'est que très récemment que les effets multiples constatés avec la DHEA (Watson & coll. *Drug*

& *Aging* 9: 274-291, 1996) ont pu être expliqués en partie par les propriétés immunostimulatrices de ses dérivés 7-hydroxylés (Morfin & Courchay *J. Steroid Biochem. Molec. Biol.* 50: 91-100, 1994; Padgett & Loria *J. Immunol.* 153: 1544-1552, 1994; Loria & coll. *J. Endocrinol.* 150: S209-S220, 1996). Les propriétés antiglucocorticoïdes présentées par la 7α- et la 7β-hydroxy-DHEA ont été prouvées et étendues à d'autres stéroïdes 7-hydroxylés comme ceux décrits dans les demandes de brevet PCT publiées sous les numéros WO 93/20687 et WO 94/08588 pour leur rôle dans le déclenchement des processus immunitaires.

Il apparait donc que la DHEA et la production de ses dérivés 7-hydroxylés diminuent avec l'âge alors que celle des glucocorticoïdes ne varie pas. Au cours du vieillissement, l'apport de stéroïdes hormonaux au niveau cutané se trouve donc modifié avec une prédominance en glucocorticoïdes dont les effets promoteurs du vieillissement cutané sont connus.

En conséquence, un apport localisé en stéroïdes 7-hydroxylés dotés d'un effet antiglucocorticoïde particulier mais naturel permet de ramener la peau traitée dans son contexte stéroïdien du jeune âge.

Or, ces propriétés n'ont jamais été décrites ou suggérées dans l'art antérieur. Ainsi, la demande de brevet PCT publiée sous le numéro WO 94/08588 ne décrit ni n'enseigne aucune application cosmétique ou dermatologique de dérivés d'hormones stéroïdes. En outre, cette demande de brevet vise des dérivés stéroïdiens dans lesquels les substitutions en position 3 et 7 indiquées dans la formule (I) ci-après sont, soit des hydroxyles, soit des fonctions esters de 1 à 10 atomes de carbone.

La demande de brevet Européen publiée sous le numéro 415 766 décrit l'utilisation d'agents rétinoïdes pour combatre l'atrophie cutanée par un mécanisme antiglucocorticoïde. Or, il n'existe aucune parenté de structure entre les rétinoïdes (vitamine A et ses dérivés) et les stéroïdes.

La demande de brevet Européen publiée sous le numéro 189 738 décrit l'utilisation de la déhydroépiandrostérone (DHEA) et de ses dérivés esters pour traiter le desèchement de la peau, or ces composés sont différents des stéroïdes objet de la présente invention.

La demande de brevet européen publiée sous le numéro 723 775 envisage l'utilisation du sulfate de DHEA dans des compositions cosmétiques et dermatologiques et suggère l'addition dans ces compositions d'hormones stéroïdes autres que le sulfate de DHEA, comme des androgènes, des oestrogènes, des progestagènes. Mais, comme indiqué précédemmenbt, ces stéroïdes sont dénués d'action hormonale, et leur utilisation n'a pour but que de palier aux effets hormonaux indésirables de la DHEA et du sulfate de DHEA. En outre, le sulfate de DHEA n'a aucune parenté structurale avec les stéroïdes objet de la présente invention.

L'invention est donc relative à l'utilisation, dans une composition cosmétique ou dermatologique à application topique destinée à prévenir ou traiter les manifestations du vieillissement cutané et/ou les effets d'irradiations UV sur la peau, d'un composé 7α ou 7β substitué de la DHEA ou de la PREG, réduits ou non en position 5, et donc répondant à la formule :

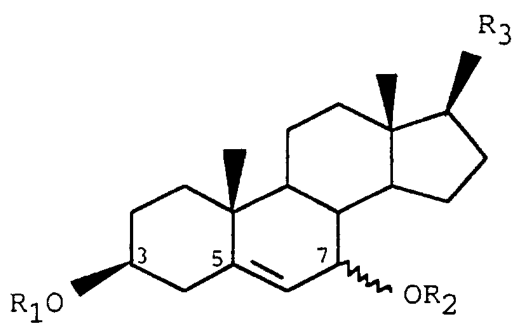

(I)

ou à la formule :

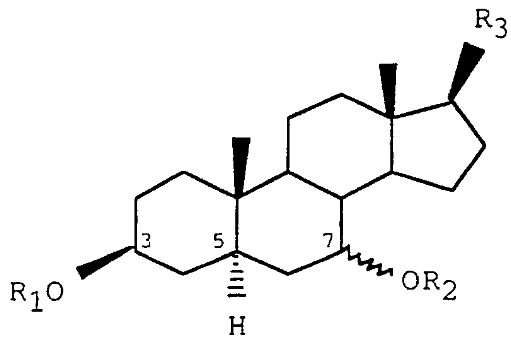

(II)

dans lesquelles :

$R_1$ est choisi parmi : un atome d'hydrogène, les fonctions ester d'acide organique de 1 à 24 atomes de carbone, ester sulfurique ou ester phosphorique, ou ether carboné de 1 à 24 atomes de carbone comprenant zéro ou plusieurs atomes d'azote, les ethers d'hydrates de carbone de 3 à 100 atomes de carbone et leurs dérivés dont ceux comprenant ou non un ou plusieurs atomes d'azote.

$R_2$ est choisi parmi : un atome hydrogène ou une fonction ester d'acide gras de 1 à 24 atomes de carbone.

$R_3$ est choisi parmi : un atome d'hydrogène, un groupe -OH, les groupes de formules : $-CO-R_4$, $-CHOH-R_4$, $=CH-CH_3$, $=COH-CH_3$, $-CHR_4-CH_3$, =O, dans lesquels $R_4$ est un groupe alcoyle comprenant de 1 à 10 atomes de carbone, de préférence méthyle, substitué ou non.

Les composés de l'invention sont des dérivés 7α ou 7β substitués de la DHEA ou de la PREG et plus particulièrement encore des dérivés 7α ou 7β-hydroxylés réduits ou non en position 5.

Un groupe de composés préférés de l'invention sont les dérivés 7α-hydroxylés, c'est à dire ceux dans lesquels l'oxygène porté dans la position 7 est axial (7α) et le substituant $R_2$ est un hydrogène .

Un autre groupe de composés préférés de l'invention sont ceux où $R_1$ est l'hydrogène, notamment la 7α-hydroxy-DHEA et la 7α-hydroxy-isoandrostérone où $R_3$ est une cétone (=O).

Il convient de remarquer que les dérivés de l'invention dans lesquels $R_1$ est un acide organique présentent une liposolubilité accrue qui offre l'avantage d'améliorer la rétention de ces composés dans les cellules, notamment au niveau des membranes et par conséquent de prolonger leur activité et leur effet sur les cellules cutanées. Parmi ces dérivés, on préfère ceux dans lesquels $R_1$ est un palmitate, un oléate ou un férulate, et notamment le 3β-palmitoyl-7ξ-hydroxy-DHEA, le 3β-oleyl-7ξ-hydroxy-DHEA et le 3β-feruloyl-7ξ-hydroxy-DHEA.

Les compositions cosmétiques ou dermatologiques de l'invention peuvent comprendre ou un ou plusieurs dérivés de stéroïde selon l'invention, ainsi que d'autres composés connus pour leur propriété cosmétologique ou dermatologique comme des hormones, et, bien entendu, les adjuvants ou véhicules classiquement utilisés dans ces domaines.

Pour l'utilisation d'un dérivé de stéroïde dans une composition cosmétique destinée à compenser, traiter et/ou prévenir les effets cutanés du vieillissement et/ou les effets d'irradiations UV sur la peau, ledit dérivé est administré à une dose comprise entre 0,05 et 10 mg par application et par jour et de préférence entre 0,05 et 5 mg par application et par jour.

L'effet de restauration ou de prévention du vieillissement cutané chez les personnes d'un certain âge ainsi que des effets protecteurs vis-à-vis des UV est applicable pour tout traitement visant à restaurer le tonus cutané, rafermir la peau et effacer les rides.

De par leur nature, les dérivés peuvent être mis en oeuvre sous des formes galéniques très diverses pour leur administration percutanée. Il peut s'agir de formes résultant de l'addition aux dérivés de l'invention de composés acceptables en cosmétique et permettant de réaliser des crèmes, des pâtes, des gels, des lotions, des émulsions "eau dans l'huile" ou "huile dans l'eau" ainsi que des formes composées de liposomes de micelles simples ou mixtes ou autres promoteurs de pénétration tels les lysophospholipides, les cyclodextrines, du polyéthylène glycol, des tensioactifs, des alcools, des acides gras, des huiles végétales. Cette liste n'est pas limitative et toute autre présentation connue de l'homme peut être envisagée dès lors qu'elle est adaptée aux dérivés stéroïdiens de l'invention qui ont comme caractéristique d'être à la fois hydrosolubles et liposolubles. Ainsi, les compositions cosmétiques ou dermatologiques peuvent se présenter sous forme de crèmes, lotions, gels et pommades ou tout autre forme généralement utilisées pour des applications topiques.

D'autres avantages et caractéristiques de l'invention apparaîtront à la lecture des exemples qui suivent, donnés à titre non limitatif, et montrant les performances obtenues par les dérivés comme agents antiapoptotiques, antiradicalaires et promoteurs de la prolifération de cellules cutanées humaines.

Exemple 1 : Effets du 3β,7α-dihydroxy-5-androstene-17-one (7α-hydroxy-DHEA) et du 3β,7α-dihydroxy-5α-androstane-17-one (7α-hydroxy-ISOA) sur l'apoptose cellulaire induite par les glucocorticoïdes.

Le thymus de souris C57BL/6 agées de 4 semaines est prélevé. La culture des thymocytes est réalisée pendant 6 heures en milieu RPMI 1640 et en présence ou en l'absence du stéroïde testé. L'apoptose (fragmentation de l'ADN) est mesurée par cytométrie de flux après marquage par l'iodure de propidium. Le phénomène apoptotique est contrôlé par électrophorèse de l'ADN révélée par le bromure d'éthidium selon la technique classique (observation d'échelles de 200 paires de bases). Les résultats rapportés dans le tableau I ci-dessous ont été obtenus:

Tableau I

| Stéroïdes dans le milieu (dans 10ml d'éthanol) | Cellules apoptotiques (%) |
|---|---|
| Ethanol seul | 41,5 |
| Dexaméthasone $10^{-6}$M | 72,7 |
| Dexaméthasone $10^{-6}$M + DHEA $10^{-6}$M | 39,0 |
| Dexaméthasone $10^{-6}$M + 7α-hydroxy-DHEA $10^{-6}$M | 58,8 |
| Dexaméthasone $10^{-6}$M + 7α-hydroxy-ISOA $10^{-6}$M | 72,0 |
| Dexaméthasone $10^{-5}$M | 73,5 |
| Dexaméthasone $10^{-5}$M + DHEA $10^{-5}$M | 51,4 |
| Dexaméthasone $10^{-5}$M + 7α-hydroxy-DHEA $10^{-5}$M | 48,6 |
| Dexaméthasone $10^{-5}$M + 7α-hydroxy-ISOA $10^{-5}$M | 46,3 |

Il apparaît de ces essais que les 7α-hydroxystéroïdes testés ont un effet antiapoptotique s'opposant à celui de la dexaméthasone sur les cellules T de souris. Leur effet à $10^{-5}$M est supérieur à celui de leur stéroïde présurseur (la DHEA ou déhydroépiandrostérone ou 3β-hydroxy-5-androstène-17-one).

Exemple 2 : Effets de la 3β,7α-dihydroxy-5-androstene-17-one (7α-hydroxy-DHEA) sur la viabilité de kératinocytes humains en culture.

Des kératinocytes humains sont obtenus à partir de pièces chirurgicales et sont cultivés en monocouche jusqu'à préconfluence. La 7α-hydroxy-DHEA est administrée à ces cultures à diverses concentrations en solution éthanolique (10 %), chaque concentration étant testée en octuple. Des contrôles sont effectués avec l'éthanol seul (10%). Après 24 heures, la viabilité des kératinocytes est mesurée par test au MTT (3-(4,5-dimethyl thiazol-2-yl)-2,5 diphenyl tetrazolium bromide) où la succinate déshydrogénase mitochondriale transforme le MTT en cristaux bleus de formazan solubles dans le DMSO (Mosmann, *J. Immunol. Methods* 65: 55-63, 1983). Les résultats des essais sur la viabilité des kéranocytes sont rapportés dans le tableau II ci-après. La viabilité cellulaire est calculée selon la formule :

$$\% \text{ viablilité} = DO_{540} \text{ produit x } 100/DO_{540} \text{ témoin.}$$

Toute valeur supérieure à 100 indique un produit favorisant la viabilité cellulaire.

Tableau II

| Stéroïdes dans le milieu (dans 10 % d'éthanol) | Viabilité des kératinocytes (%) |
|---|---|
| 10 % d'éthanol seul (témoin) | 100 |
| 7α-hydroxy-DHEA $10^{-4}$M | 124 ± 10 |
| 7α-hydroxy-DHEA $5.10^{-5}$M | 111 ± 7 |
| 7α-hydroxy-DHEA $10^{-5}$M | 119 ± 7 |
| 7α-hydroxy-DHEA $5.10^{-6}$M | 147 ± 9 |
| 7α-hydroxy-DHEA $10^{-6}$M | 154 ± 6 |

Tableau II (suite)

| Stéroïdes dans le milieu (dans 10 % d'éthanol) | Viabilité des kératinocytes (%) |
|---|---|
| 7α-hydroxy-DHEA $5.10^{-7}$M | 139 ± 3 |
| 7α-hydroxy-DHEA $10^{-7}$ M | 147 ± 5 |
| 7α-hydroxy-DHEA $10^{-8}$M | 127 ± 3 |

Ces résultats montrent que la 7α-hydroxy-DHEA augmente significativement la viabilité des kératinocytes humains aux concentrations entre $10^{-4}$M et $10^{-8}$M, le maximum (augmentations entre 54% et 39% de la viabilité) étant obtenu entre $5.10^{-6}$M et $10^{-7}$M. Par ailleurs, aucune cytotoxicité n'a été observée. D'autres tests comparatifs ont démontré que le précurseur DHEA était sans effet (100 ± 5).

<u>Exremple 3 : Effets de la 3β,7α-dihydroxy-5-androstene-17-one (7α-hydroxy-DHEA) sur la prolifération de fibroblastes humains en culture.</u>

Les cultures de fibroblastes humains (femme de 32 ans) sont ensemencées en plaques 24 puits à raison de 50 000 cellules/puits dans le milieu de culture standard (DMEM, gentamycine, amphotéricine B, penicilline, L-glutamine, 10% SVF). Les essais sont effectués sur 4 séries de 3 puits. Après 24 h, les fibroblastes adhèrent au support et 3 séries sont traitées par la 7α-hydroxy-DHEA aux concentrations de $10^{-6}$M, $5.10^{-6}$M et $10^{-7}$M. La quatrième série ne contient que le vecteur (éthanol). Les milieux sont renouvelés quotidiennement, et à 96 h (72 h de contact de la 7α-hydroxy-DHEA à l'essai), les fibroblastes sont comptés sur cellule de Malassez en présence de bleu trypan.

Les résultats des effets sur la prolifération des fibroblastes sont rapportés dans le tableau III ci-dessous.

Tableau III

| Stéroïdes dans le milieu | Nombre de Fibroblastes | Augmentation de la viabilité (%) |
|---|---|---|
| Témoin | 190 667 ± 6 766 | / |
| 7α-hydroxy-DHEA $10^{-7}$M | 230 667 ± 8 511 | + 21 |
| 7α-hydroxy-DHEA $10^{-6}$M | 268 000 ± 27 154 | + 41 |
| 7α-hydroxy-DHEA $5.10^{-6}$M | 258 667 ± 3 351 | + 36 |

Ces résultats démontrent que, dans les conditions expérimentales, le traitement des fibroblastes par la 7α-hydroxy-DHEA à $10^{-7}$M, $10^{-6}$M et $5.10^{-6}$M augmente la prolifération cellulaire respectivement de 21%, 41% et 36% par rapport aux fibroblastes témoins non traités.

<u>Exemple 4 : Effet anti-radicalaire du 3β,7α-dihydroxy-5-androstène-17-one (7α-hydroxy-DHEA) sur un suspension de kératinocytes humains.</u>

Des kératinocytes provenant d'un donneur sain (femme de 25 ans) sont cultivés jusqu'au stade subconfluent en milieu spécifique (KGM) pour la prolifération des kératinocytes. Les suspensions obtenues sont réparties en triplicata dans 4 séries dont 3 sont irradiées pendant 30 min avec une lampe émettant des UVA afin d'activer la production de radicaux libres. Parmi les trois séries irradiées, une contient les vitamines C+E (0,7%) et sert de référence de protection, une contient la 7α-hydroxy-DHEA à $10^{-6}$M et la dernière sert de contrôle. Le tableau IV ci-après rapporte la mesure des effets anti-radicallaires.

Les radicaux libres produits génèrent des peroxydes lipidiques qui sont dosés par chemiluminescence (Belghmi & coll. *J. Biolum. Chemilum.* <u>2</u>: 113-119, 1982). L'efficacité de la 7α-hydroxy-DHEA est calculée sur la base des témoins non irradiés et de la référence de protection.

Tableau IV

| Kératinocytes | Chemiluminescence | Efficacité |
|---|---|---|
| Témoins non irradiés | 2 529 ± 153 | / |
| Témoins irradiés | 427 750 ± 137 322 | / |
| Irradiés + 0,7% Vit. C+E | 2 970 ± 288 | 100% |
| Irradiés + 7α-hydroxy-DHEA $10^{-6}$M | 44 164 ± 13 303 | 90% |

Dans les conditions de cette étude, l'efficacité antiradicalaire *in vitro* de la 7α-hydroxy-DHEA à $10^{-6}$M est de

90%. La 7α-hydroxy-DHEA peut être considérée comme un bon produit antiradicalaire.

## Revendications

1. Utilisation pour la fabrication d'une composition dermatologique pour prévenir ou traiter les manifestations du vieillissement cutané et/ou les effets d'irradiations UV sur la peau, d'un composé répondant à la formule :

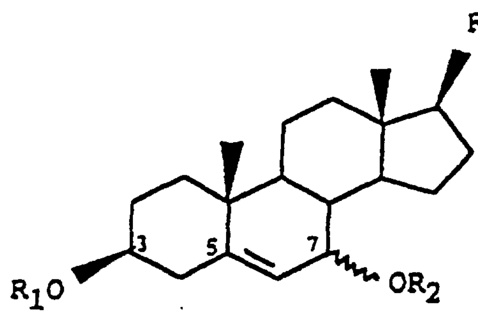

(I)

ou à la formule :

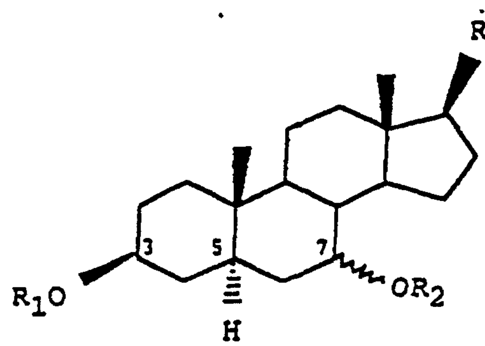

(II)

dans lesquelles :

$R_1$ est choisi parmi : un atome d'hydrogène, les fonctions ester d'acide organique de 1 à 24 atomes de carbone, ester sulfurique ou ester phosphorique, ou ether carboné de 1 à 24 atomes de carbone comprenant zéro ou plusieurs atomes d'azote, les ethers d'hydrates de carbone de 3 à 100 atomes de carbone et leurs dérivés comprenant ou non un ou plusieurs atomes d'azote.
$R_2$ est choisi parmi : un atome hydrogène ou une fonction ester d'acide gras de 1 à 24 atomes de carbone.
$R_3$ est choisi parmi : un atome d'hydrogène, un groupe -OH, les groupes de formules : $-CO-R_4$, $-CHOH-R_4$, $=CH-CH_3$, $=COH-CH_3$, $-CHR_4-CH_3$, =O, dans lesquels $R_4$ est un groupe alcoyle comprenant de 1 à 10 atomes de carbone substitué ou non.

2. Utilisation d'un composé de formules (I) ou (II), selon la revendication 1, dans lesquelles $R_2$ et/ou $R_1$ est un atome d'hydrogène.

3. Utilisation d'un composé de formules (I) ou (II), selon l'une des revendications 1 ou 2, dans lesquelles $R_3$ est une cétone.

4. Utilisation d'un composé de formules (I) ou (II), selon l'une quelconque des revendications 1 à 3, dans lesquelles $R_1$ est une fonction ester d'acide gras choisi parmi un oléate, un palmitate, un férulate.

5. Utilisation selon la revendication 2, **caractérisée en ce que** le composé de formule (I) ou (II) est de la 7α-hydroxy-DHEA.

**6.** Utilisation selon la revendication 3,
**caractérisée en ce que** le composé de formule (I) ou (II) est la 7α-hydroxy-isoandrostérone.

**7.** Utilisation selon la revendication 4, **caractérisée en ce que** le composé de formule (I) ou (II) est choisi parmi le 3β-palmitoyl-7ξ-hydroxy-DHEA, le 3β-oleyl-7ξ-hydroxy-DHEA et le 3β-feruloyl-7ξ-hydroxy-DHEA.

**8.** Utilisation selon l'une quelconque des revendications précédentes, **caractérisée en ce que** la composition contient au moins un composé de formule (I) ou (II) associé à un ou plusieur adjuvants ou véhicules utilisés en cosmétologie ou dermatologie.

**9.** Utilisation selon l'une quelconque des revendications précédentes, **caractérisée en ce que** la composition contient entre 0,05 et 10 mg et de préférence entre 0,05 et 5 mg d'un composé de formule (I) ou (II).

**10.** Procédé de traitement cosmétique des manifestations du vieillissement cutané et/ou des effets non thérapeutiques d'irradiations UV sur la peau, comprenant l'application sur la peau d'une composition cosmétique contenant au moins un composé répondant à la formule :

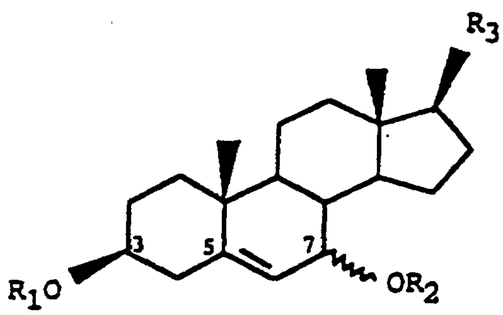

(I)

ou à la formule :

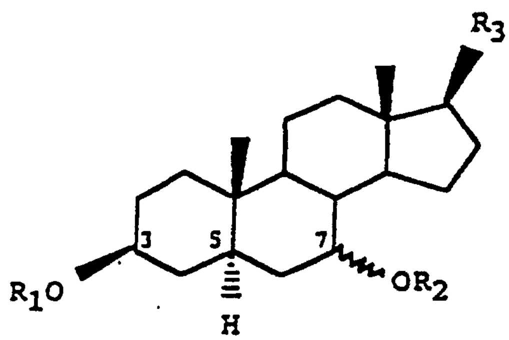

(II)

dans lesquelles :

$R_1$ est choisi parmi : un atome d'hydrogène, les fonctions ester d'acide organique de 1 à 24 atomes de carbone, ester sulfurique ou ester phosphorique, ou ether carboné de 1 à 24 atomes de carbone comprenant zéro ou plusieurs atomes d'azote, les ethers d'hydrates de carbone de 3 à 100 atomes de carbone et leurs dérivés comprenant ou non un ou plusieurs atomes d'azote.
$R_2$ est choisi parmi : un atome hydrogène ou une fonction ester d'acide gras de 1 à 24 atomes de carbone.
$R_3$ est choisi parmi : un acome d'hydrogène, un groupe -OH, les groupes de formules : $-CO-R_4$, $-CHOH-R_4$, $=CH-CH_3$, $=COH-CH_3$, $-CHR_4-CH_3$, =O, dans lesquels $R_4$ est un groupe alcoyle comprenant de 1 à 10 atomes de carbone substitué ou non.

**11.** Procédé selon la revendication 10, **caractérisé en ce qu'**il comprend l'application sur la peau d'une dose de ladite composition comprise entre 0,05 et 10 mg par application et par jour et de préférence entre 0,05 et 5 mg par application et par jour.

**12.** Utilisation dans une composition cosmétique pour prévenir ou traiter les manifestations du vieillissement cutané et/ou les effets d'irradiations non thérapeutiques UV sur la peau, d'un composé répondant à la formule :

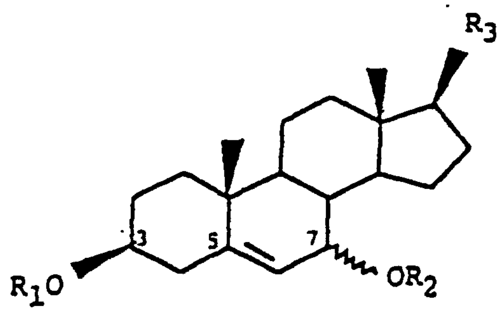

(I)

ou à la formule :

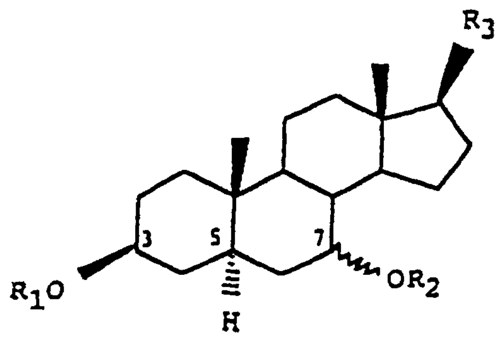

(II)

dans lesquelles :

$R_1$ est choisi parmi : un atome d'hydrogène, les fonctions ester d'acide organique de 1 à 24 atomes de carbone, ester sulfurique ou ester phosphorique, ou ether carboné de 1 à 24 atomes de carbone comprenant zéro ou plusieurs atomes d'azote, les ethers d'hydrates de carbone de 3 à 100 atomes de'carbone et leurs dérivés comprenant ou non un ou plusieurs atomes d'azote.

$R_2$ est choisi parmi : un atome hydrogène ou une fonction ester d'acide gras de 1 à 24 atomes de carbone.

$R_3$ est choisi parmi : un atome d'hydrogène, un groupe -OH, les groupes de formules : -CO-$R_4$, -CHOH-$R_4$, =CH-$CH_3$, =COH-$CH_3$, -$CHR_4$-$CH_3$, =O, dans lesquels $R_4$ est un groupe alcoyle comprenant de 1 à 10 atomes de carbone substitué ou non.

## Patentansprüche

**1.** Verwendung einer Verbindung der Formel

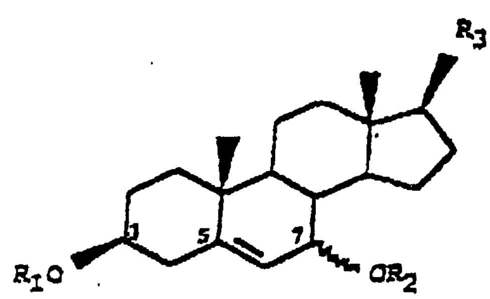

(I)

oder der Fomel

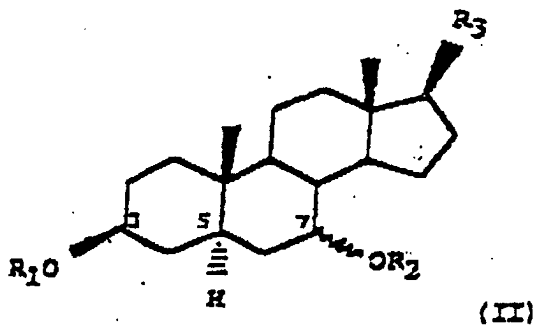

(II)

worin:

$R_1$ ausgewählt ist aus: einem Wasserstoffatom, den Esterfunktionen einer organischen Säure mit 1-24 Kohlenstoffatomen. dem Schwefelsäureester oder Phosphorsäureester oder Kohlenstoffethern mit 1-24 Kohlenstoffatomen, die kein oder mehrere Stickstoffatome enthalten, den Kohlenhydratethern mit 3-100 Kohlenstoffatomen und ihren Derivaten, die kein oder mehrere Stickstoffatome enthalten;

$R_2$ ausgewählt ist aus: einem Wasserstoffatom und einer Esterfunktion einer Fettsäure mit 1-24 Kohlenstoffatomen;

$R_3$ ausgewählt ist aus: einem Wasserstoffatom, einer Gruppe -OH, den Gruppen der Formeln $-CO-R_4$, $-CHOH-R_4$, $=CH-CH_3$, $=COH-CH_3$, $-CHR_4-CH_3$, =O, in denen $R_4$ eine substituierte oder nicht-substituierte Alkoylgruppe ist, die 1-10 Kohlenstoffatome umfasst;

zur Herstellung einer dermatologischen Zusammensetzung zur Prävention oder Behandlung der Erscheinungsformen der Hautalterung und/oder der Wirkung von UV-Bestrahlung auf der Haut.

**2.** Verwendung einer Verbindung der Formel (I) oder (II) nach Anspruch 1, in denen $R_2$ und/oder $R_1$ ein Wasserstoffatom sind/ist.

**3.** Verwendung einer Verbindung der Formel (I) oder (II) nach Anspruch 1 oder Anspruch 2, in denen $R_3$ ein Keton ist.

**4.** Verwendung einer Verbindung der Formel (I) oder (II) nach einem der Ansprüche 1-3, in denen $R_1$ eine Esterfunktion einer Fettsäure ist, die aus einem Oleat, einem Palmitat, einem Ferulat ausgewählt ist.

**5.** Verwendung nach Anspruch 2,
**dadurch gekennzeichnet,**
**dass** die Verbindung der Formel (I) oder (II) 7α-Hydroxy-DHEA ist.

**6.** Verwendung nach Anspruch 3,
**dadurch gekennzeichnet,**
**dass** die Verbindung der Formel (I) oder (II) 7α-Hydroxyisoandrosteron ist.

**7.** Verwendung nach Anspruch 4,
**dadurch gekennzeichnet,**
**dass** die Verbindung der Formel (I) oder (II) unter 3β-Palmitoyl-7ξ-Hydroxy-DHEA, 3β-Oleyl-7ξ-Hydroxy-DHEA und 3β-Feruloyl-7ξ-Hydroxy-DHEA ausgewählt ist.

**8.** Verwendung nach einem der vorangehenden Ansprüche,
**dadurch gekennzeichnet,**
**dass** die Zusammensetzung mindestens eine Verbindung der Formel (I) oder (II), assoziiert mit einem oder mehreren Adjuvanzien oder Vehikeln, die in der Kosmetik oder Dermatologie verwendet werden, enthält.

**9.** Verwendung nach einem der vorangehenden Ansprüche, **dadurch gekennzeichnet,** **dass** die Zusammensetzung 0,05-10 mg und vorzugsweise 0,05-5 mg einer Verbindung der Formel 1 oder 2 enthält.

**10.** Verfahren zur kosmetischen Behandlung der Erscheinungsformen der Hautalterung und/oder der nichttherapeutischen Wirkungen von UV-Bestrahlung auf der Haut, umfassend Auftragen einer kosmetischen Zusammensetzung auf die Haut, welche mindestens eine Verbindung der Formel

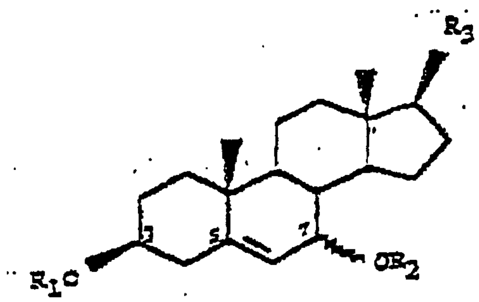

(I)

oder der Formel

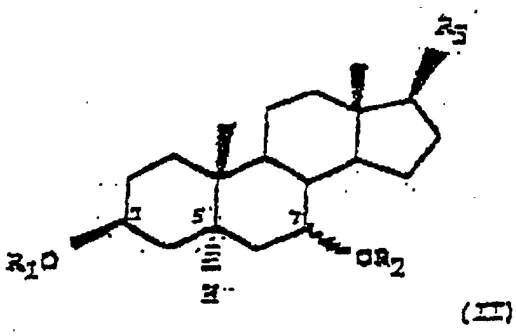

(II)

enthält, worin:

$R_1$ ausgewählt ist aus: einem Wasserstoffatom, den Esterfunktionen einer organischen Säure mit 1-24 Kohlenstoffatomen, dem Schwefelsäureester oder Phosphorsäureester oder den Kohlenstoffethern mit 1-24 Kohlenstoffatomen, die kein oder mehrere Stickstoffatome enthalten, den Kohlenhydratethern mit 3-100 Kohlenstoffatomen und ihren Derivaten, die kein oder mehrere Stickstoffatome enthalten;

$R_2$ ausgewählt ist aus: einem Wasserstoffatom und einer Esterfunktion einer Fettsäure mit 1-24 Kohlenstoffatomen;

$R_3$ ausgewählt ist aus: einem Wasserstoffatom, einer Gruppe -OH, den Gruppen der Formeln $-CO-R_4$, $-CHOH-R_4$, $=CH-CH_3$, $=COH-CH_3$, $-CHR_4-CH_3$, =O, in denen $R_4$ eine substituierte oder nicht-substituierte Alkoylgruppe ist, die 1-10 Kohlenstoffatome umfasst.

**11.** Verfahren nach Anspruch 10, **dadurch gekennzeichnet,** **dass** es *das* Auftragen einer Dosis der genannten Zusammensetzung, die 0,05-10 mg pro Anwendung und pro Tag und vorzugsweise 0,05-5 mg pro Anwendung und pro Tag enthält, umfasst.

**12.** Verwendung einer Zusammensetzung der Formel:

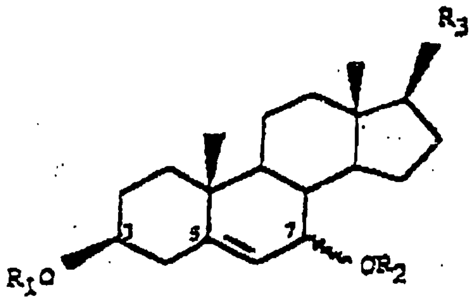

(I)

oder der Formel:

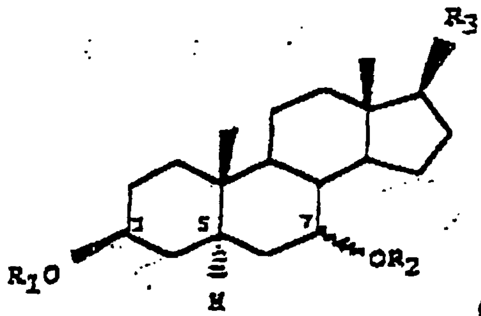

(II)

worin:

$R_1$ ausgewählt ist aus; einem Wasserstoffatom, den Esterfunktionen einer organischen Säure mit 1-24 Kohlenstoffatomen, dem Schwefelsäureester oder Phosphorsäureester oder den Kohlenstoffethern mit 1-24 Kohlenstoffatomen, die kein oder mehrere Stickstoffatome enthalten, den Kohlenhydratethern mit 3-100 Kohlenstoffatomen und ihren Derivaten, die kein oder mehrere Stickstoffatome enthalten;

$R_2$ ausgewählt ist aus: einem Wasserstoffatom und einer Esterfunktion einer Fettsäure mit 1-24 Kohlenstoffatomen;

$R_3$ ausgewählt ist aus: einem Wasserstoffatom, einer Gruppe -OH, den Gruppen der Formeln $-CO-R_4$, $-CHOH-R_4$, $=CH-CH_3$, $=COH-CH_3$, $-CHR_4-CH_3$, $=O$, in denen $R_4$ eine substituierte oder nicht-substituierte Alkoylgruppe ist, die 1-10 Kohlenstoffatome umfasst;

in einer kosmetischen Zusammensetzung zur Prävention oder Behandlung der Erscheinungsformen der Hautalterung und/oder der Effekte nichttherapeutischer UV-Bestrahlung auf der Haut.

## Claims

**1.** Use for the manufacture of a dermatological composition to avert or treat the manifestations of skin ageing and/or the effects of UV irradiation on the skin, of a compound corresponding to the formula:

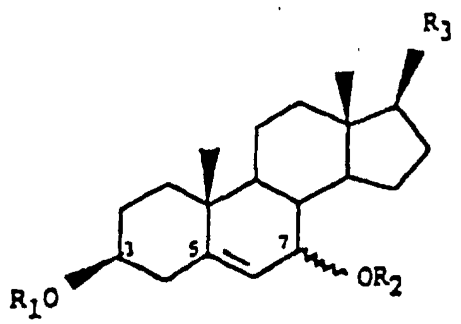

(I)

or the formula:

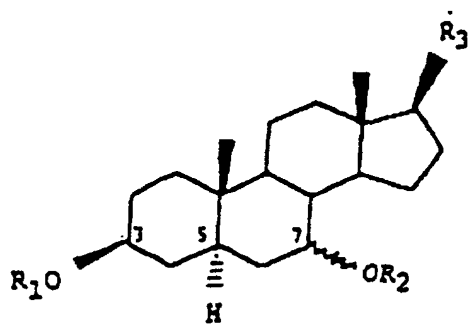

(II)

in which:

$R_1$ is chosen from among; a hydrogen atom, organic acid ester groups with 1 to 24 carbon atoms, sulphuric ester or phosphoric ester, or carbon-containing ether with 1 to 24 carbon atoms comprising zero or several nitrogen atoms, carbohydrate ethers with 3 to 100 carbon atoms and their derivatives with or without one or several nitrogen atoms.

$R_2$ is chosen from among: a hydrogen atom or a fatty acid ester group of 1 to 24 carbon atoms.

$R_3$ is chosen from among: a hydrogen atom, an -OH group, groups with the formulae: $-CO-R_4$, $-CHOH-R_4$, $=CH-CH_3$, $=COH-CH_3$, $-CHR_4-CH_3$, = in which $R_4$ is a substituted or unsubstituted alkyl group comprising 1 to 10 carbon atoms.

**2.** Use of a compound composed of formula (I) or (II), according to claim I, in which $R_1$ and/or $R_2$ is a hydrogen atom.

**3.** Use of a compound composed of formula (I) or (II), according to one of the claims 1 or 2, in which $R_3$ is a ketone.

**4.** Use of a compound composed of formula (I) or (II), according to any one of the claims 1 to 3, in which $R_1$ is a fatty acid ester group chosen from an oleate, palmitate or ferulate.

**5.** Use according to claim 2, **characterized in that** the compound of formula (I) or (II) is 7α-hydroxy-DHEA.

**6.** Use according to claim 3, **characterized in that** the compound of formula (I) or (II) is 7α-hydroxy-isoandrosterone.

**7.** Use according to claim 4, **characterized in that** the compound of formula (I) or (II) is chosen from among 3β-palmitoyl-7ξ-hydroxy-DHEA, 3β-oleyl-7ξ-hydroxy-DHEA and 3β-feruloyl-7ξ-hydroxy-DHEA.

**8.** Use according to any one of the preceding claims, **characterized in that** the composition contains at least one compound of formula (I) or (II) combined with one or more adjuvants or vehicles used in cosmetology or dermatology.

**9.** Use according to any one of the preceding claims, **characterized in that** the composition contains between 0.05 and 10 mg and preferably between 0.05 and 5 mg of a compound of formula (I) or (II).

**10.** A procedure of cosmetic treatment of the manifestations of skin ageing and/or the effects of non-therapeutic UV irradiation on the skin, comprising the application to the skin of a cosmetic composition containing at least one compound corresponding to the formula:

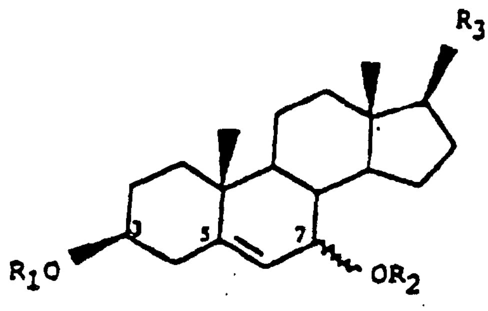

(I)

or to the formula:

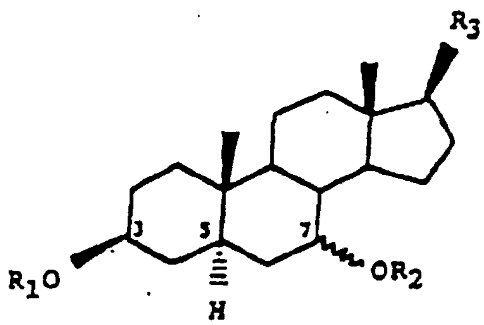

(II)

in which:

$R_1$ is chosen from among; a hydrogen atom, organic acid ester groups with 1 to 24 carbon atoms, sulphuric ester or phosphoric ester, or carbon-containing ether with 1 to 24 carbon atoms comprising zero or several nitrogen atoms, carbohydrate ethers with 3 to 100 carbon atoms and their derivatives with or without one or several nitrogen atoms.
$R_2$ is chosen from among: a hydrogen atom or a fatty acid ester group with 1 to 24 carbon atoms.
$R_3$ is chosen from among: a hydrogen atom, an -OH group, groups having the formula: $-CO-R_4$, $-CHOH-R_4$, $=CH-CH_3$, $=COH-CH_3$, $-CHR_4-CH_3$, =O, in which $R_4$ is a substituted or unsubstituted alkyl group comprising 1 to 10 carbon atoms.

**11.** A procedure according to claim 10, **characterized in that** it comprises the application to the skin of a dose of the said composition amounting to between 0.05 and 10 mg per application and per day and preferably between 0.05 and 5 mg per application and per day.

**12.** Use in a cosmetic composition to avert or treat the manifestations of skin ageing and/or the effects of non-therapeutic UV irradiation on the skin, of a compound corresponding to the formula:

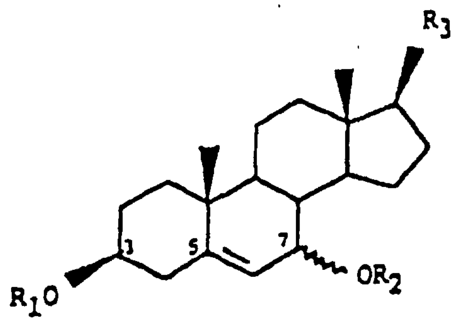

(I)

or the formula:

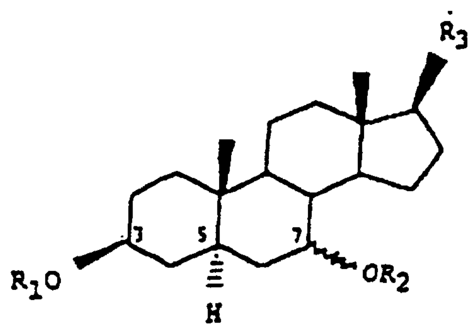

(II)

in which:

$R_1$ is chosen from among; a hydrogen atom, organic acid ester groups with 1 to 24 carbon atoms, sulphuric ester or phosphoric ester, or carbon-containing ether with 1 to 24 carbon atoms comprising zero or several nitrogen atoms, carbohydrate ethers with 3 to 100 carbon atoms and their derivatives with or without one or several nitrogen atoms.

$R_2$ is chosen from among: a hydrogen atom or a fatty acid ester group with 1 to 24 carbon atoms.

$R_3$ is chosen from among: a hydrogen atom, an -OH group, groups with the formulae: $-CO-R_4$, $-CHOH-R_4$, $=CH-CH_3$, $=COH-CH_3$, $-CHR_4-CH_3$, $=O$, in which $R_4$ is a substituted or unsubstituted alkyl group comprising 1 to 10 carbon atoms.